# EUROPEAN PATENT SPECIFICATION

(11) **EP 1 266 629 B1**
(45) Date of publication and mention of the grant of the patent: **29.11.2006**
(21) Application number: 02253790.6
(22) Date of filing: 30.05.2002
(51) Int. Cl.: A61B 17/12, A61M 25/00, A61M 25/10

(54) **Delivery system using balloon catheter**
Ablagesystem unter Verwendung eines Ballonkatheters
Système d'introduction utilisant un cathéter à ballonnet

(30) Priority: 11.06.2001 US 878530
(43) Date of publication of application: 18.12.2002
(73) Proprietor: Cordis Neurovascular, Inc., Miami Lakes, Florida 33014 (US)
(72) Inventor: Dominguez, Larry, West Miami, Florida 33144 (US)
(74) Representative: Belcher, Simon James

(56) References cited:
- EP-A- 0 664 104
- WO-A-02/45597
- DE-U- 29 506 307
- US-A- 5 810 787
- US-A- 5 921 958
- US-A- 5 957 940

## Description

This invention relates to a balloon catheter. The preferred embodiment of the invention is a novel method for placing embolic coils within an aneurysm.

The use of embolic coils placed within an aneurysm for treating the aneurysm is well known. Various devices are known for delivering the embolic coils through the patient's vessel to the aneurysm. Typically these embolic coils, which generally take the form of helically wound coils or random wound coils, are coupled to a coil deployment device which serves to introduce the coils into the aneurysm and then enables release of the coils through various types of release mechanisms. It has been found to be difficult to place the coils in the exact position desired because of the relative lack of stability of the deployment device within the vessel during the introduction of the embolic coil to the aneurysm.

WO-02/45597 which forms part of the prior art under Article 54(3), relates to an intravascular balloon catheter. The catheter has a combined guidewire/delivery lumen. The balloon comprises a multi-lobed design with a delivery hole disposed adjacent to a lobe of the balloon, or between adjacent balloon lobes.

EP-A-0664104 discusses a balloon catheter. The catheter comprises a lumen for inflating a balloon, a delivery lumen coupled to an exit port and a lumen for a guidewire.

It has been used for the delineation of claim 1 in the two-part form.

The present invention provides a delivery catheter that can be utilized to deliver guidewires, embolics, diagnostic, and therapeutic agents via a delivery lumen.

Accordingly, in one aspect, the invention provides a balloon catheter which comprises:
a catheter body which has a proximal end and a distal end and which defines an inflation lumen, and a delivery lumen separate from the inflation lumen;
a balloon adjacent the distal end;
an inflation port at the proximal end, which communicates via the inflation lumen with the balloon;
a delivery port at the proximal end;
a guidewire opening at the distal end;
a side opening adjacent the distal end, spaced from the guidewire opening;
in which the balloon is radially aligned with said side opening and wholly oppositely positioned on the catheter with respect to the side opening. The guidewire opening and the side opening both communicate with the delivery lumen.

The catheter can be used in a method which comprises the steps of providing a catheter having a proximal end and a distal end, a balloon adjacent to the distal end, and an inflation port at the proximal end communicating via an inflation lumen with a balloon. A guidewire opening is provided at the distal end and a spaced, side opening is provided adjacent to the distal end. The catheter is introduced into the vessel of a patient via a guidewire which extends through the guidewire opening to generally align the side opening with the location to be treated. The balloon is inflated to stabilize the position of the catheter. A medical agent is thereafter introduced from the proximal end of the catheter and through the side opening to deliver the medical agent to the location within the patient's vessel to be treated. Thereafter, the balloon is deflated and the catheter is withdrawn from the patient's vessel.

The catheter can be used to place an embolic coil at a location within an aneurysm. The catheter is introduced into the vessel of a patient using a guidewire extending through the guidewire opening to generally align the side opening with the aneurysm. The balloon is inflated to stabilize the position of the catheter and an embolic coil deployment device is introduced to the proximal end of the catheter through the side opening via a delivery lumen to delivery an embolic coil into the aneurysm. Once the desired number of embolic coils are delivered into the aneurysm, the balloon is deflated and the catheter is thereafter withdrawn from the patient's vessel.

The catheter can be preloaded with a guidewire extending from the delivery port through the delivery lumen and distal of the guidewire opening.

A hydraulic deployment system can be utilized for delivering a medical agent using the catheter of the invention, for example to deliver an embolic coil to an aneurysm. The hydraulic deployment device includes a positioning catheter having a distal tip for retaining medical agent. When the positioning catheter is pressurized with a fluid, the distal tip expands outwardly to release the agent at the preselected position. The methods disclosed in this application do not form part of the present invention.

Embodiments of the present invention will now be described by way of example with reference to the accompanying drawings, in which:
Fig. 1 is a partial sectional view of a delivery catheter constructed in accordance with the principles of the present invention;
Fig. 2 is a partial sectional view of a vascular occlusive coil deployment system that can be used with the catheter of Fig. 1;
Fig. 3 is a diagrammatic view the delivery catheter of Fig. 1 in use to deliver an embolic coil to an aneurysm;
Fig. 4 is a cross-sectional view of the catheter of Fig. 3, taken along the plane of the line 4-4' of Fig. 3;
Fig. 5 is a cross-sectional view of the catheter of Fig. 3, taken along the plane of the line 5-5' of Fig. 3;
Fig. 6 is a cross-sectional view of the catheter of Fig. 3, taken along the plane of the line 6-6' of Fig. 3;
Figs. 7 to 10 are diagrammatic sequential views of a method of placing embolic coils in accordance with an embodiment of the present invention.

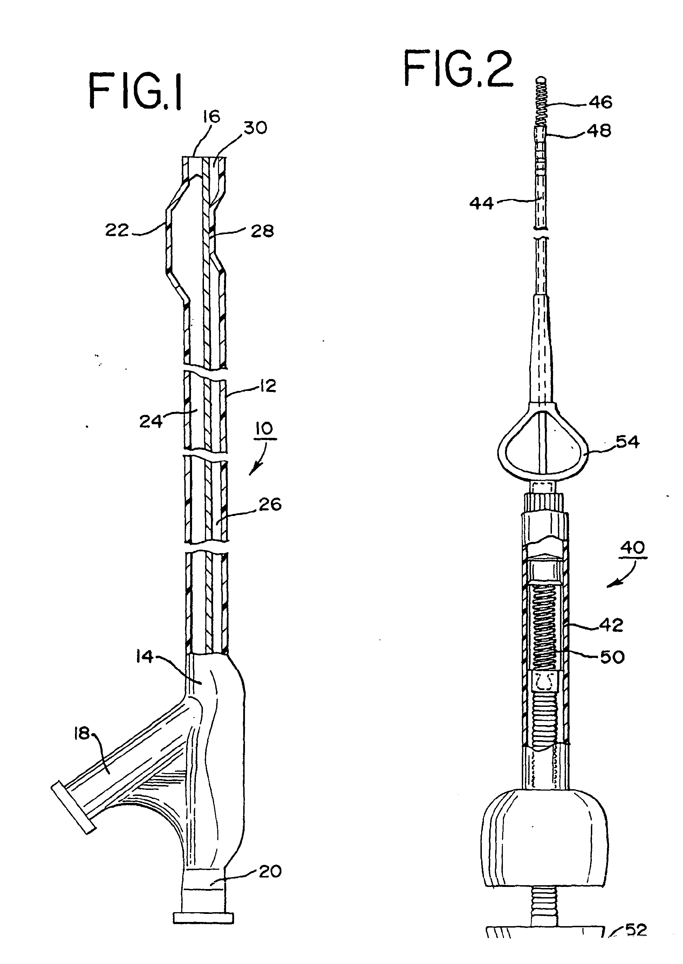

Referring to the drawings, Figs. 1 and 3 to 6 show a balloon catheter 10 includes a tubular catheter body 12 having a proximal end 14 and a distal end 16. At the proximal end 14 there is an inflation port 18 and a delivery port 20. A flexible balloon 22 is located adjacent distal end 16 and is in fluid communication with an inflation lumen 24, which is in fluid communication with inflation port 18. A secondary port (not shown) may also be carried by the catheter and may be in fluid communication with inflation lumen 24. The secondary port, and inflation lumen 24 could be used to purge air trapped in the balloon and body.

The catheter body and balloon are preferably formed of one or more polymers. As an example, the proximal portion of the catheter may be formed from nylon and the remainder of the catheter to its distal end may be formed of polyurethane. The inflatable balloon 22 may be formed from silicone material although it is to be understood that various other balloon materials and other materials known for forming a catheter may be used.

Catheter body 12 also defines a delivery lumen 26 which communicates with delivery port 20. Catheter body 12 also has a side opening 28 which is oppositely and radially aligned with balloon 22 and is in communication with delivery lumen 26.

Also in communication with delivery lumen 26 is a guidewire opening 30 which opening 30 is axially aligned with the catheter body 12 and is generally perpendicular to side opening 28.

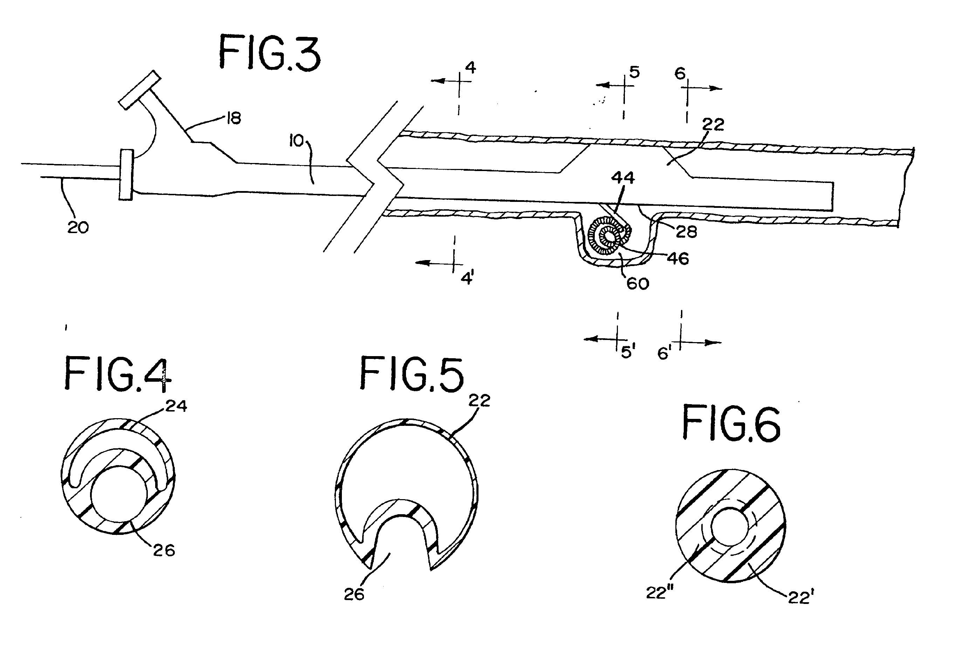

Fig. 5 shows a cross-section of balloon 22 when it is fully inflated; Fig. 6 shows, as reference numeral 22', the balloon with a reduced diameter and as indicated by reference numeral 22", the original inner diameter of the balloon.

In Fig. 2, there is illustrated a deployment device 40 for embolic coils.

Although the catheter is described above in relation to the deployment of embolic coils, it is to be understood that the catheter can be used for the deployment of other medical agents, including liquid embolic agents, biocompatible polymer-solvent combinations, biocompatible polymers, and other embolizing compositions as are known in the art. Further, the medical agent that is deployed could be a diagnostic agent or a therapeutic agent. Although it will be apparent from the description how other medical agents may be deployed pursuant to the present invention, since an embolic coil is a preferred embodiment the description of the invention will be primarily referenced to the deployment of embolic coils.

The deployment device 40 of Fig. 2 includes a syringe 42, coupled to the proximal end of a catheter 44. An embolic coil 46 is disposed within the lumen of the distal end 48 of the catheter. The proximal end of the coil 46 is tightly held within the lumen of the distal section 48 of catheter 44 until the deployment system is activated for release of the coil. Syringe 42 includes a threaded piston 50 which is controlled by handle 52 for infusing fluid into the interior of the catheter 44. Catheter 44 includes a winged hub 54 which aids in the insertion of the catheter into the delivery port 20 of catheter 10.

Embolic coil 46 may take various forms and configurations. Its proximal end is within distal end 48 of catheter 44, which distal end 48 is flexible to form a fluid type seal with the proximal end of coil 46. When a hydraulic pressure is applied by piston 50 to the interior of catheter 44, the distal section 48 begins to expand radially to release coil 46 from the distal end 44 and to deploy coil 46 at the desired location, for example within an aneurysm.

It is to be understood that various types of deployment devices may be used including but not limited to those operating electrically, mechanically, adhesively, magnetically, etc. and coil 46 may take numerous forms as is well know in the art.

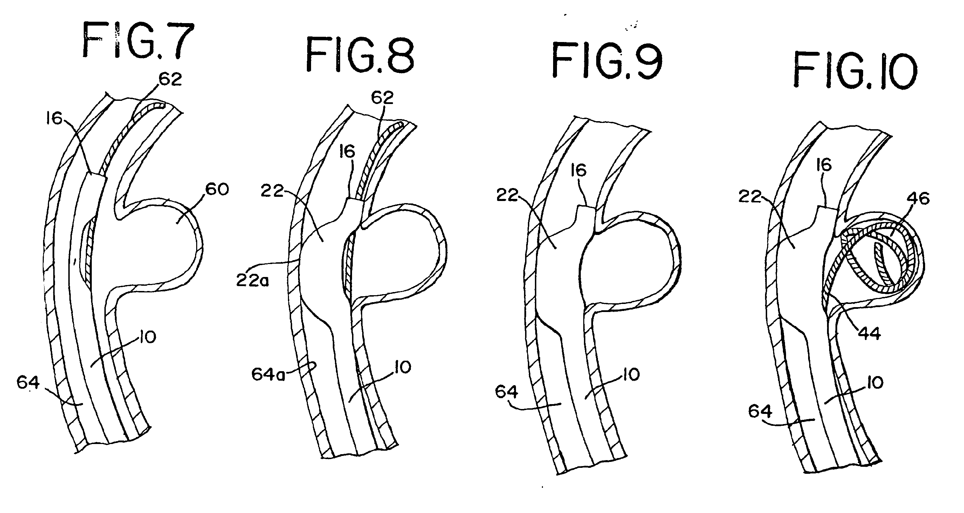

In accordance with the present invention, catheter 10 is utilized to enable the stable delivery of embolic coils to an aneurysm. Fig. 3 is a diagrammatic view of catheter 10 enabling the delivery of a coil 46 to a brain aneurysm 60 through side port 28 of the catheter 10. The method for placing the embolic coil 46 at a location within aneurysm 60 is illustrated, in sequence, in Figs. 7 to 10.

Referring to Fig. 7, catheter 10, which has previously been preloaded with a guidewire 62, is introduced into the patient's vessel 64. Guidewire 62 is preloaded to extend through delivery port 20, delivery lumen 26 and guidewire opening 30. The distal end of guidewire 62 is fed through a vessel, followed by catheter 10 containing guidewire 62, so that opening 28 will be aligned adjacent the aneurysm 60. Once so aligned, as illustrated in Fig. 8, balloon 22 will be inflated by providing inflation fluid via inflation lumen 24 to balloon 22 with the outer tip 22a of balloon 22 being compressed against the inner wall 64a of the vessel 64. This will serve to stabilize the catheter 10 and maintain its location within the vessel.

Once catheter 10 is in position with side opening 28 aligned with the aneurysm 60 and the balloon 22 compressed against the vessel wall, guidewire 62 is withdrawn via delivery port 20 and a catheter such as catheter 44, connected at its proximal end to a deployment device such as device 40, is inserted via delivery port 20 into delivery lumen 26. When the embolic coil 46 reaches side port 28, it will exit side port 28 into the aneurysm 60. When the coil has been placed in the desired location with the aneurysm, the coil is then released from the deployment device and the deployment device is withdrawn via the delivery port 20.

While balloon 22 remains compressed against the vessel wall, another embolic coil may be attached to the catheter 44 of the delivery device 40. The catheter 44 of the delivery device 10 is again inserted into the delivery port 20 and through the delivery lumen 26 to side port 28 so that another embolic coil will be placed in a desired location within aneurysm 60. This process can be repeated until a desired number of coils have been placed within the aneurysm. The balloon 22 is then deflated and catheter 10 is removed from the vessel.

It can be seen that a novel system has been disclosed in which an embolic coil is securely placed within an aneurysm with a catheter that is stabilized and is relatively simple in construction and easy to use. Variations are contemplated. For example, as stated above an additional port could be used in communication with the balloon to purge air trapped in the balloon and body. Further, in addition to the delivery of embolics, the system can be utilized to delivery guidewires, diagnostics and therapeutic agents via a delivery lumen. The multiple lumen body may be composed of polymers and/or metals and a balloon may be preformed and attached to the inflation lumen adjacent the distal end of the catheter or formed from the inflation lumen of the multiple lumen body.

## Claims

1. A balloon catheter (10) which comprises:
a catheter body (12) which has a proximal end (14) and a distal end (16) and which defines an inflation lumen (24), and a delivery lumen (26) separate from the inflation lumen (24);
a balloon (22) adjacent the distal end (16);
an inflation port (18) at the proximal end (14), which communicates via the inflation lumen (24) with the balloon (22);
a delivery port (20) at the proximal end (14);
a guidewire opening (30) at the distal end (16);
a side opening (28) adjacent the distal end, spaced from the guidewire opening (30); **characterised in that**
the balloon (22) is radially aligned with said side opening (28) and wholly oppositely positioned on the catheter (10) with respect to the side opening (28); and
the guidewire opening (30) and the side opening (28) both communicate with the delivery lumen (26).

2. A catheter system, which comprises a catheter (10) as claimed in claim 1, and a hydraulic deployment device for displacing a medical agent to be delivered using the catheter (10) from the delivery lumen (26) thereof.

3. A catheter system, which comprises a catheter as claimed in claim 1, and a deployment device (40) for delivering an embolic coil (46) through the side opening (28).

## Patentansprüche

1. Ballonkatheter (10), welcher umfaßt:
einen Katheterkörper (12), welcher ein proximales Ende (14) und ein distales Ende (16) aufweist, und welcher ein Inflationslumen (24) und ein zu dem Inflationslumen (24) separates Abgabelumen (26) definiert;
einen Ballon (22) nahe dem distalen Ende (16);
einen Inflationsanschluß (18) an dem proximalen Ende (14), welcher über das Inflationslumen (24) mit dem Ballon (22) kommuniziert;
einen Abgabeanschluß (20) an dem proximalen Ende (14);
eine Leitdrahtöffnung (30) an dem distalen Ende (16);
eine Seitenwandöffnung (28) nahe dem distalen Ende, die von der Leitdrahtöffnung (30) beabstandet ist;
**dadurch gekennzeichnet, daß**
der Ballon (22) mit der Seitenwandöffnung (28) radial ausgerichtet und bezüglich der Seitenwandöffnung (28) gänzlich entgegengesetzt an dem Katheter (10) positioniert ist; und
die Leitdrahtöffnung (30) und die Seitenwandöffnung (28) beide mit dem Abgabelumen (26) kommunizieren.

2. Kathetersystem, welches einen Katheter (10), wie in Anspruch 1 beansprucht, und eine hydraulische Bereitstellungsvorrichtung zum Verdrängen eines mit Hilfe des Katheters (10) abzugebenden medizinischen Wirkstoffs aus dem Abgabelumen (26) umfaßt.

3. Kathetersystem, welches einen Katheter, wie in Anspruch 1 beansprucht, und eine Bereitstellungsvorrichtung (40) zum Abgeben einer Emboliespiralfeder (46) durch die Seitenwandöffnung (28) umfaßt.

## Revendications

1. Cathéter à ballonnet (10) comprenant :
un corps de cathéter (12) qui possède une extrémité proximale (14) et une extrémité distale (16) et qui définit une lumière de gonflage (24) et une lumière de distribution (26) séparée de la lumière de gonflage (24) ;
un ballonnet (22) adjacent à l'extrémité distale (16) ;
un orifice de gonflage (18) à l'extrémité proximale (14), qui communique via la lumière de gonflage (24) avec le ballonnet (22) ;
un orifice de distribution (20) à l'extrémité proximale (14) ;
une ouverture pour fil-guide (30) à l'extrémité distale (16) ;
une ouverture latérale (28) adjacente à l'extrémité distale, espacée de l'ouverture pour fil-guide (30) ;
**caractérisé en ce que**
le ballonnet (22) est aligné radialement sur ladite ouverture latérale (28) et positionné complètement à l'opposé sur le cathéter (10) par rapport à l'ouverture latérale (28) ; et
l'ouverture pour fil-guide (30) et l'ouverture latérale (28) communiquent toutes deux avec la lumière de distribution (26).

2. Système de cathéter, comprenant un cathéter (10) selon la revendication 1, et un dispositif de déploiement hydraulique pour déplacer un agent médical devant être délivré en utilisant le cathéter (10) à partir de la lumière de distribution (26) de celui-ci.

3. Système de cathéter, comprenant un cathéter selon la revendication 1, et un dispositif de déploiement (40) pour délivrer une spire métallique d'embolisation (46) à travers l'ouverture latérale (28).
